# EUROPEAN PATENT SPECIFICATION

(11) **EP 2 001 928 B1**
(45) Date of publication and mention of the grant of the patent: **12.05.2010**
(21) Application number: 07727225.0
(22) Date of filing: 22.03.2007
(51) Int. Cl.: C08G 63/08, C08G 63/00, A61L 33/00

(54) **Use of a highly haemocompatible and biodegradable polymer**
Verwendung eines sehr gut blutverträglichen und biologisch abbaubaren Polymeren
Utilisation d'un polymère hautement hémocompatible et biodegradable

(30) Priority: 30.03.2006 US 787183 P
(43) Date of publication of application: 17.12.2008
(73) Proprietor: Universita' Degli Studi Del Piemonte Orientale "Amedeo Avogadro", 13100 Vercelli (IT)
(72) Inventor: CANNAS, Mario, I-10138 Torino (IT); RENO', Filippo, I-28100 Novara (IT)
(74) Representative: Freyria Fava, Cristina
(86) International application number: PCT/EP2007/052749
(87) International publication number: WO 2007/113124

(56) References cited:
- WO-A-03/099169
- WO-A-2005/040247
- FR-A1- 2 862 879
- RENO F ET AL: "Effect of vitamin E addition to poly(d,l)-lactic acid on surface properties and osteoblast behaviour" BIOMATERIALS, ELSEVIER SCIENCE PUBLISHERS BV., BARKING, GB, vol. 26, no. 28, October 2005 (2005-10), pages 5594-5599, XP004878237 ISSN: 0142-9612

## Description

### Field of the invention

The present invention concerns the use of a new highly haemocompatible and biodegradable polymer particularly, as a coating material for implantable prosthesis in the animal or human body.

### Background of the invention

Many biodegradable polymers are used as drug delivery systems for the coating of vascular endoprosthesis, mainly metallic stent, in order to release drugs or other agents (e.g. nucleic acids) to reduce stent thrombogenic tendency and to contrast neointima hyperplasia and consequent vascular stenosis [1], a pathobiological process that still occurs in 10% to 50% of cases currently treated [2].

One of the main challenges for this research area is to reduce the negative interactions occurring between the polymer used in the production of drug-eluting stents and the complex and fast reactive blood environment. In fact, biodegradable polymers such as polyglycolic acid/polylactic acid (PGLA) or polycaprolactone (PCL), which are considered good candidates for this kind of application on the basis of in vitro tests, after implantation have been demonstrated to induce a marked inflammation with subsequent neointimal thickening [3]. Later some other polymers were found to be biologically inert and stable for at least 6 months [4,5], and now the research is focused on the use of biomimetic substances such as phosphorylcoline [6] that does not interfere with the re-endothelization and the degree of neointimal formation. Among biodegradable polymers poly (D,L) lactic acid (P(D,L)LA), largely used in the orthopaedic field for its good mechanical property, is an interesting candidate for stent coating as it undergoes a slow scission to lactic acid in the body [7,8], but unluckily it also activates both granulocyte [9] and platelet [10].

In fact, P(D,L) LA has been recently used as a paclitaxel-eluting coronary stent with good results in inhibiting restenosis in an animal model, but the unloaded polymer induced a long lasting local inflammatory response that probably caused an underestimation of the paclitaxel effect on the restenosis [11].

Restenosis is an important tissue healing response after arterial wall injury occurring during the transluminal coronary revascularization [12]. The arterial wall response involves vessel elastic recoil, negative remodelling, thrombus formation at the site of injury, smooth muscle cell (SMC) proliferation and migration and excessive extracellular matrix production [13]. In order to reduce restenosis in the last fifteen years the use of a metallic stent has been introduced avoiding elastic recoil and negative remodelling at the site of injury [14]. Nevertheless, it has been observed in-stent restenosis in 10% to 50% of cases treated [15], a phenomenon due mainly to the SMC proliferation and migration that create the so-called neointima. In order to control neointima hyperplasia and consequent vascular stenosis, biodegradable polymers are used for metallic stent coating to deliver anti-proliferative drugs [16-18].

In the last 10 years the use of additives or drugs to improve materials biocompatibility has been widely tested. Among the various additives used, the most interesting is the Vitamin E (α-tocopherol), a potent biological and biocompatible anti-oxidant and antiinflammatory agent [19-22], widely used in the biomaterial field [23-26]. Interestingly all the biological processes involved in the tissue response to stent implantation (mainly inflammation and smooth muscle cell proliferation) can be modulated directly by Vitamin E [19-22].

An example of the use of polylactic acid as a drug delivery system is provided i.a. in WO-A-2005/053768, wherein the use of polylactic acid as a matrix loaded with pentoxyfylline and an anti-oxidant agent (i.a. tocopherol acetate) is disclosed, particularly in connection with the use of polylactic acid as a coating material for implantable endoprosthesis in order to release at the implantation site the necessary active agents.

### Summary of the invention

Object of the present invention is the use of a new highly haemocompatible and biodegradable polymer which overcomes the disadvantages of the prior art, as a coating material for implantable prosthesis, preferably heart valves, stents.

Such objects are achieved thanks to the solution claimed in the ensuing claims, which form integral part of the technical teaching of the invention herein provided.

The present invention concerns the use of a new biodegradable polymer composed by poly(D,L)lactic acid (PDLLA) and Vitamin E (α-tocopherol), named Polylactil-E. This polymer shows a high degree of haemocompatibility compared to the original polymer (PDLLA) and it is a good candidate for the coating of different biomaterials. In particular, Polylactil-E can be used for the coating of endovascular prosthesis in order to reduce the adhesion of platelet and granulocyte, the formation of thrombi and the inflammatory response to the endoprosthesis implantation.

In a further embodiment, the new biodegradable polymer of the present invention can be used a drug delivery system for local administration of pharmaceutically active compounds, which can act i.a. as anti-restenosis agents.

### Brief description of the drawings

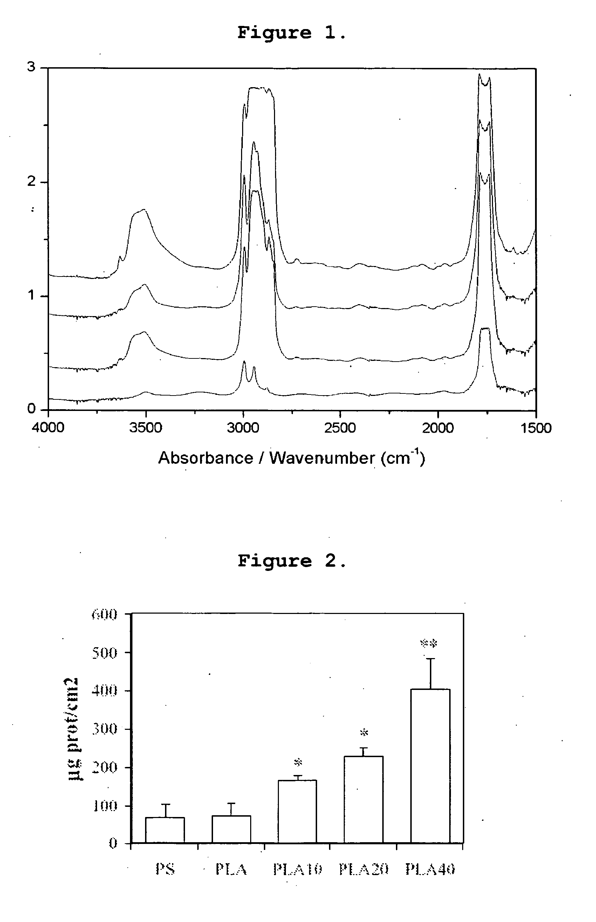

- **Figure 1****.** FTIR spectra of control P(D,L)LA and P(D,L)LA enriched with 10%, 20% and 40% (w/w) Vit. E.
- **Figure 2****.** Quantification of protein adsorbed onto polystyrene (PS), control P(D,L)LA (PLA) and P(D,L)LA enriched with 10% (PLA10) , 20.%(PLA20) and 40% (PLA40) Vit.E. *p<0.05, **p<0.001, compared to control P(D,L)LA.

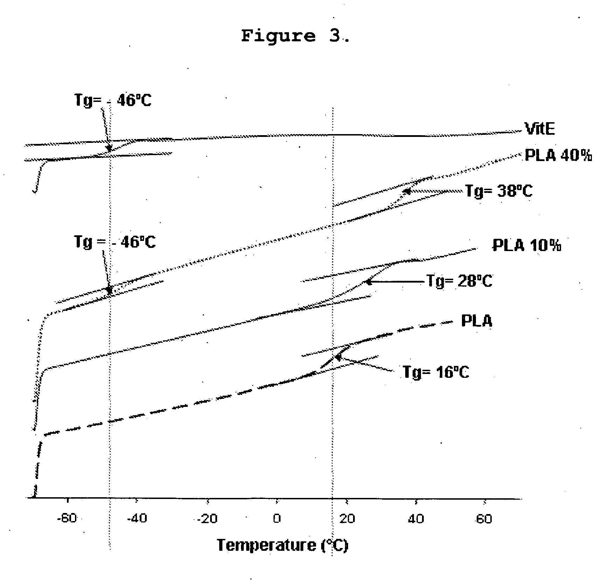

- **Figure 3****.** DSC thermograms of P(DL)LA (PLA) vit.E , 10%Vit.E/PLA and 40% Vit.E /PLA (Polylactil-E)

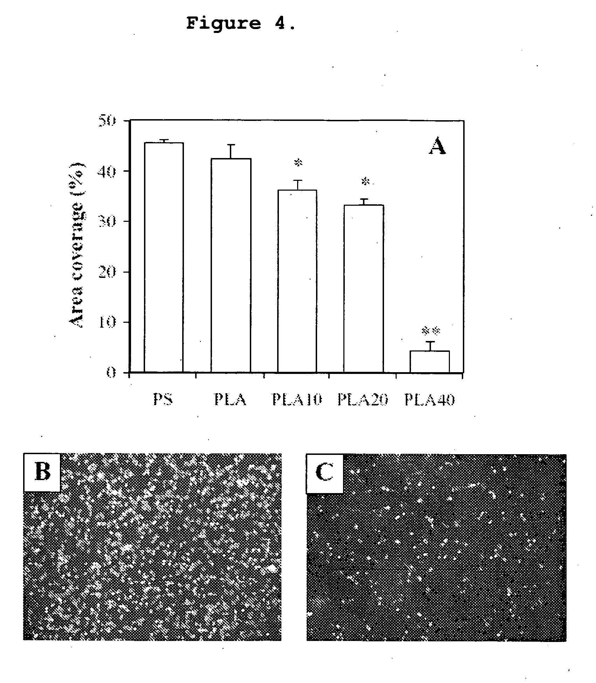

- **Figure 4.** A) Quantification of platelet adhesion expressed as % of area coverage obtained from the fluorescence emitted by platelet stained with phalloidine-TRIC and adherent onto polystyrene (PS), control P(D,L)LA (PLA) and P(D,L)LA enriched with 10% (PLA10) , 20% (PLA20) and 40% (PLA40) Vit.E after 0.5 hour of incubation.. B) Platelet adhesion onto P(D,L)LA and C) PLA40. Magnification = 40X

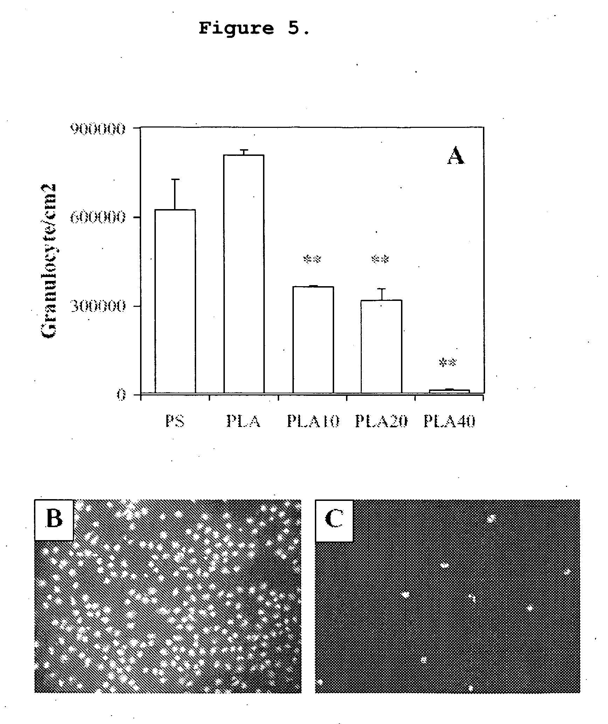

- **Figure 5.** A) Quantification of granulocyte adhesion expressed as cellular count of adherent granulocytes stained with Acridine Orange (AO) and scored onto PS, PLA and P(D,L)LA enriched with Vit.E (PLA10, 20, 40) after 1 hour incubation. Adherent cells were counted in 10 different fields per sample at 10X magnification and their number was expressed as adherent granulocytes/cm² B) Granulocyte adhesion onto P(D,L)LA and C) PLA40. *p<0..05, **p<0.001, compared to control P(D,L)LA.

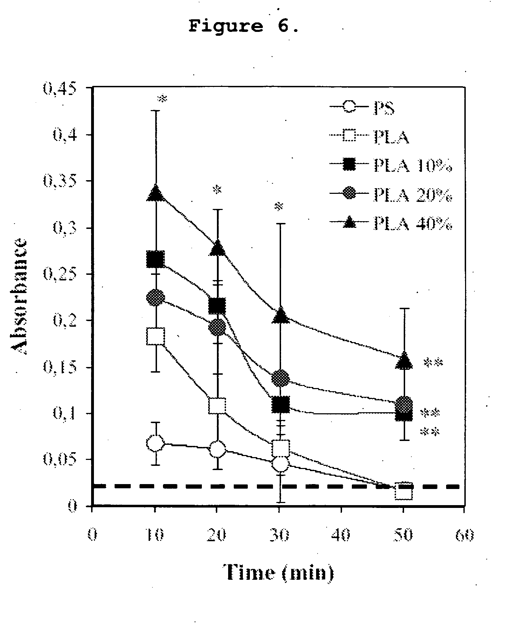

- **Figure 6****.** Absorbance of haemolysed haemoglobin solutions obtained after contact with PS disks, P(D,L)LA and Vit.E-enriched P(D,L)LA films versus time of contact.

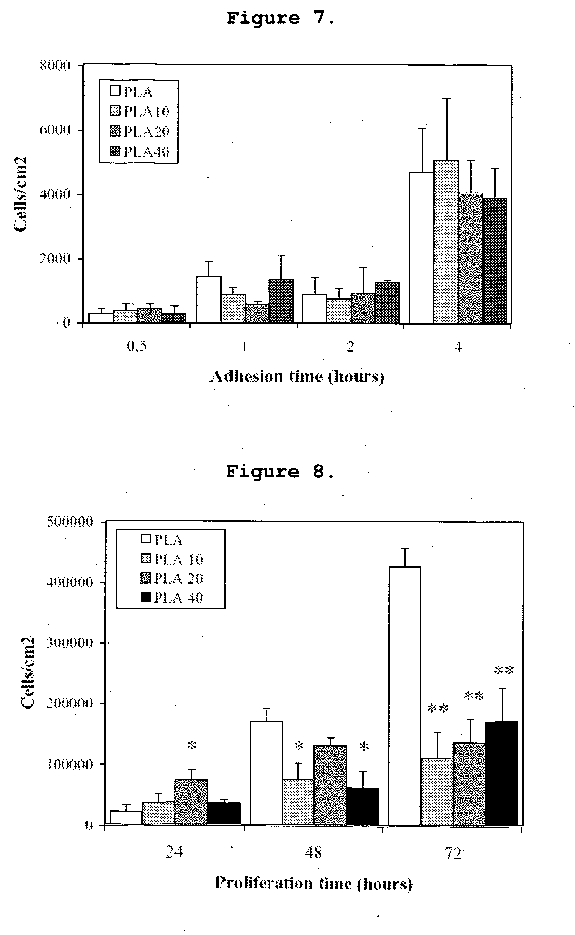

- **Figure 7****.** Quantification of A10 cell adhesion expressed as cellular count of adherent cell stained with Acridine Orange (AO) and scored onto control P(D.L)LA (PLA) and P(D,L)LA enriched with Vit.E (PLA10, 20, 40) after 0.5, 1,2 and 4 hours of incubation. Adherent cells were counted in 10 different fields per sample at 10X magnification and their number was expressed as adherent cells/cm² ± standard deviation (S.D.).
- **Figure 8****.** Quantification of A10 cell adhesion expressed as cellular count of adherent cell stained with Acridine Orange (AO) and scored onto control P(D. L)LA (PLA) and P(D, L)LA enriched with Vit.E (PLA10, 20,30) after 24, 48 and 72 hours. Adherent cells were counted in 10 different fields per sample at 10X magnification and their number was expressed as adherent cells/cm² ± standard deviation (S.D.). *p<0.05, **p<0.001 compared to PLA.

### Detailed description of the invention

The invention will now be described in detail in relation to some preferred embodiments by way of not limiting examples.

The present inventors enriched P(D,L)LA films with Vitamin E (at various concentrations) obtaining a new kind of polymer (named Polylactil-E) with different physical and biological characteristics compared to the normal P(D,L)LA. The physical and biological behaviour of Polylactil-E makes this polymer suitable for improving surface haemocompatibility of different kinds of implantable biomaterials (e.g. metal stent).

In the following examples the preparation of Polylactil E, its physical and biological behaviours will be described.

### Example 1 - Preparation and physical characterization of Polylactil-E.

### Polylactil-E preparation

P(D,L)LA (100% D,L, average mol wt 75, 000-120, 000) and Vitamin E (α-tocopherol) were purchased from Sigma-Aldrich (Milwaukee, WI, USA). P(D,L)LA was dissolved under gentle shacking in chloroform (99.8% pure, Sigma Aldrich) at a concentration of 0.05 g/ml (5% solution w/v). Vitamin E ((±) α-tocopherol, synthetic 95% pure HPLC) was dissolved 1:1 (v/v) in ethanol (absolute extrapure, Merck, Darmstadt, Germany) (**differente da lavoro su Biomaterials**) and then added to the P(D,L)LA/chlorophorm in order to obtain a solution with 10-40% Vit.E (w/w) (highest Vit.E concentration added = 20 mg/ml final solution). After 10 min shaking the solution was sprayed onto glass dishes at a pressure of 2 atm and the solvent was evaporated at room temperature under vacuum for 3 hours in the dark. The operation was repeated to form film sheets of ∼1 mm thickness. Films were then cut under sterile conditions into square samples (∼1 cm²) and stored at 4°C for no more than 1 week.

### FTIR analysis

The presence of Vitamin E in the films obtained was assessed using Fourier transformed infrared spectroscopy (FTIR). FTIR spectra for polymers surfaces were obtained at 4 cm⁻¹ resolution using a Bruker IFS 113v spectrophotometer, equipped with MCT cryodetector. The spectra for IR analysis were executed on thin transparent films of control and Vit.E P(D,L)LA (area=1cm² surface). IR spectra were executed in transmission and recorded in the region of mid infrared at a nominal temperature of ∼400 K. FTIR analysis. In Fig.1 the FTIR absorbance spectra of P(D,L)-LA and P(D,L)-LA enriched with Vit. E at various concentrations recorded in the region of 4000-1500 cm⁻¹ are shown.

The band at ∼3500 cm⁻¹ indicates the stretching of O-H and it is present in every sample, as OH groups are present in both P(D,L)LA and Vit.E structures. Two bands at ∼4000 cm⁻¹ represent the stretching of -CH₃. The -CH₃ functions are also P(D,L)LA and Vit.E structures, but in the Vit.E there are both aromatic and aliphatic -CH₃, the former emitting at slightly higher frequencies than the latter. The intensity of bands at 3500 and 4000 cm⁻¹ increased with the Vit.E concentration added to the P(D,L)LA, indicating the dose-dependent Vit.E presence.

### Wettability test.

Contact angle measurements were carried out in order to evaluate the wettability of the Vit.E-enriched P(D,L)LA films. An equal volume of distilled water (100 µl) was placed on every sample by means of a micropipette, forming a drop or spreading on the surface. Photos were taken through lenses (LEITZ IIA optical stage microscope equipped with LEICA DFC320 video-camera) to record drop images. Measure of the contact angle was performed by analyzing drop images (3 for each samples) using Scion Image software. In the wettability test performed using the control P(D,L)LA surface, a distilled water drop put on the polymer surface formed an angle of almost 90° (89,6° ± 1,5°), while the Vit.E addition decreased the water contact angle starting from 10% concentration (water contact angle = 62,3°±1,5°, p<0.001). The 20% Vit.E P(D,L)LA wettability (water contact angle = 58,2°±1,9°) was not significantly higher than the one measured for 10%Vit.E films, while wettability increased significantly for 40% Vit. E samples (water contact angle = 49,4°±2,3°).

### Protein adsorption.

Protein adsorption assay was performed in triplicate using human plasma pool obtained from 10 healthy donors. Blood (10 ml) was centrifuged at 200 x g for 10 minutes to obtain platelet rich plasma (PRP) . PRP was then centrifuged at 1600 x g for 10 minutes to separate platelet. Plasma was then stored at -20°C prior to use. PS disks, P(D,L)LA and P(D,L)LA/Vit.E films (1cm²) were covered with 200 µl of undiluted human plasma pool and incubated for 1 hour at 37°C. At the end of incubation, plasma was removed and films were washed three times with PBS. Adsorbed proteins were collected by incubating samples with 1 ml of 2% sodium dodecyl sulfate (SDS) solution in PBS for 4 hours at room temperature and under vigorous shaking. Amount of adsorbed proteins was measured in triplicate using a commercial protein quantification kit (BCA, Pierce, Rockford, IL). The sample optical density was read at 562 nm against a calibration curve created using bovine serum albumin (BSA, 25-2000 µg/ml). The results were expressed as micrograms of total protein adsorbed for cm²±standard deviation (S.D).

As expected from the results of wettability test, protein adsorption assay evidenced that addition of Vit.E to P(D,L)LA induced a higher total protein adsorption compared to control P(D,L)LA (70±32,9. µg/cm²) and cell culture grade polystyrene (PS, 66, 3±34, 1 µg/cm²) (Fig.2) . In fact the adsorbed protein quantity measured for 10%, 20% and 40% Vit.E P(D,L)LA was respectively 162±6,9, 226±22,5 and 400,7±52,5 µg/cm².

### Measurement of the Glass Transition Temperature (Tg) for Vit.E, PDLLA and PDLLA/Vit. E 10 and 40% (Polylactil-E).

The physical characteristics observed in the Vit.E enriched P(D,L)LA suggested a more deep interaction between the polymer and the Vitamin E. Therefore, Vit.E, P(D,L)LA and P(D,L)LA/Vit.E 10 and 40% (Polylactil-E) have been studied by differential scanning calorimetry (DSC). Briefly, accurately weighed samples (∼10 mg) of the different materials were placed in aluminum DSC pans and sealed. The lids were vented with a pinhole in the center. To determine the Tg of the various samples, they were first cooled to -100°C and then scanned from -100 to 100°C at 20°C/min. The DSC thermograms obtained were examined and the midpoints of the baseline shifts were taken as glass transition temperatures. As shown in Figure 3 the Vit.E was present in the 40% Vit.E/P(D,L) LA (Polylactil-E) in a free form (Tg=-46°C) and its presence altered the Tg of P(D,L)LA in a dose-dependent and saturable fashion, suggesting the creation of a kind of binding between the linear polymer and the Vitamin E.

### Example 2 - Biological activity of Polylactil-E.

### a) Haemocompatibility

### Granulocyte and platelet adhesion

Platelets and granulocytes were obtained from human peripheral venous blood (20 ml) obtained from 10 healthy donors (age range = 20-36) using EDTA as anticoagulant. All the blood samples were used within 3 hours from sampling. Granulocytes were separated from whole blood using a modification of the method of Boyum [27]. Blood (10ml) was layered onto a Ficoll-Hypaque density gradient and centrifuged for 20 minutes at 2000 rpm to separate mononuclear cells from erythrocytes and granulocytes. The mononuclear fraction was discharged and erythrocytes were then lysed using an ammonium chloride lysing solution (150mM NH₄Cl, 10mM NaHCO₃, 1 mM EDTA, pH7.4) for 20 minutes at 4°C. Pellet containing granulocytes was then centrifuged twice in sterile phosphate buffer (PBS), cells were counted in optical microscopy using trypan blue exclusion test (viability >98%) and suspended at a concentration of 1x10⁶ cells/ml in RPMI 1640 (Euroclone, Milan, Italy) medium supplemented with 10% heat-inactivated fetal calf serum (Euroclone, Milan, Italy) containing penicillin (100 u/ml), streptomycin (100mg/ml) and L-glutamine (2 mM) (Euroclone, Milan, Italy) in polypropilene tubes. Granulocyte suspension (200 µl) was seeded onto cell culture grade polystyrene disks (area ∼1cm2) and P(D,L)LA and P (D, L) LA/Vit.E films (area=1cm²) and incubated for 1 hour in a humidified atmosphere containing 5% CO₂ at 37°C.

In order to obtain platelets, whole blood (10 ml) was centrifuged at 200 x g for 10 minutes to obtain platelet rich plasma (PRP). PRP was then centrifuged at 1600 x g for 10 minutes to separate platelet then resuspended in 10 ml RPMI 1640 medium supplemented with 10% heat-inactivated fetal calf serum (Euroclone, Milan, Italy). Aliquots of platelet suspension (200 µl) were seeded onto PS disks, P(D,L)LA and P(D,L)LA/Vit.E films and incubated for 0.5 hour in a humidified atmosphere containing 5% CO₂ at 37°C.

Cell counting and morphological analysis of adherent platelet and granulocyte were performed using a fluorescence microscope Aristoplan Leitz equipped with a digital camera Leica DFC320. At the end of the incubation time adherent platelet and granulocyte were washed three times with cold PBS (pH 7.4) and fixed for 15 minutes at room temperature using a solution of formaldehyde (3.7%) and sucrose (3%) in PBS (pH 7.4). Platelets were treated for 5 minutes with Triton X-100 solution (2% vol/vol) in PBS and stained with 0.1 µM phalloidin-TRIC (Sigma-Aldrich, Milwaukee, WI USA) for 1h at 37°C. Platelets adhesion was measured as surface coverage with phalloidin-stained platelets (% area coverage ± standard deviation - S.D.) by measuring the fluorescence presence in 10 different fields for sample observed using a 10X magnification. Fluorescence presence was measured using Leika QWin software.

Adherent granulocytes were stained for 10 minutes at room temperature in the dark with a 0.2% solution of Acridine Orange (AO) and counted in 10 different fields per sample at 10X magnification. Scoring was performed by three separate observers, blind to the sample treatment using Leika QWin software and expressed as adherent granulocyte/cm² ± standard deviation (S.D.). Platelet morphology was observed using a 40X magnification while granulocytes were observed using a 25X magnification.

In vitro platelet adhesion testing was performed to study the quantity and the morphology of adherent platelets onto control P(D,L)LA and Vit.E-enriched P(D,L)LA. As shown in Fig.4A the percentage of area covered by platelet adherent onto PS and P(D,L)LA was 45,5±0,6% and 42,3± 2.9% respectively. Platelet adhesion slightly decreased onto 10% and 20% Vit.E P(D,L)LA films (36,1±2,0% and 34,4±1,4% respectively, p<0,05 compared to control P(D,L)LA), even if no statistically significant difference was observed as regards the percentages of covered area measured for the two Vit.E-enriched polymers. Besides platelet adhesion dropped dramatically onto 40%Vit.E P(D,L)LA films where only 4,4±1,7% of the polymer area was covered by platelet (p<0.001). Platelet morphology was altered by the presence of high Vit.E concentration as observed by the actin staining with phalloidin. In fact as shown in Fig.4B adherent platelet observed onto control P(D,L)LA formed aggregates and 50-70% of platelet showed a spread morphology, while the few adherent platelet observed onto 40%Vit.E P(D,L)LA films (Fig.4C) were mostly isolated and their morphology was mainly roundish.

As shown in Fig.5A granulocytes adhered both to PS (619200 ± 104840 cells/cm²) and P(D,L)LA (806400 ± 17900 cells/cm²) after 1 hour incubation. The Vit.E presence in P(DL)LA films strongly decreased granulocyte adhesion at 10% (360400 ± 4500 cells/cm² , p<0.001) and 20% Vit.E (317000 ± 37200 cells/cm², p<0.001). Also for granulocyte adhesion no statistically significant differences were observed between 10% and 20% Vit.E polymer films, and also in this case the presence of 40% Vit.E reduced the cell adhesion (11100 ± 2890 cells/cm², p<0.001).

Granulocyte adherent to P(D,L)LA and stained with AO showed the typical polylobate nucleus and a spread morphology observed for activated granulocyte (Fig.5B), while the few adherent granulocyte observed onto 40%Vit.E P(D,L)LA films (Fig.5C) showed a roundish morphology with a lower cellular size compared to the granulocyte adherent onto control P(D,L)LA.

### Clotting time

The tromboresistant properties of the P(D,L)LA and Vit.E-enriched P(D,L)LA films were evaluated using fresh human blood using the kinetic clotting method [28] . For this test, 100 µl of fresh blood were taken directly from the plastic syringe used for the blood collection and immediately dropped onto the film specimens and onto polystyrene disks (PS). After a predetermined contact time (10, 20, 40 and 50 minutes), specimens were transferred into plastic tubes each containing 20 ml of distilled water and incubated for 5 minutes. The surface ability to induce blood clotting was deduced by the quantity of free haemoglobin measurable at every time point. In fact, the red blood cells that had not been trapped in a thrombus were haemolysed and the concentration of free haemoglobin dispersed in water was measured by monitoring the absorbance at 540 nm. The absorbance values were plotted versus the blood contacting time and the clotting times were derived using optical density curves. Each absorbance value represents the average of 10 measurements ± S.D. In Fig.6 the blood clotting profile for PS, - P(D,L)LA and Vit.E P(D,L)LA films are shown. The absorbance of the haemolyzed haemoglobin solution varied with time, and the higher the absorbance, the better the thromboresistence. The present inventors indicated as clotting time the time at which the absorbance equals 0.02. PS was able to reduce quickly haemoglobin absorbance and PS samples coagulated completely after 45-47 minutes. P(D,L)LA samples showed a similar clotting time, but the coagulation process seemed to occur more slowly compared to PS. The addition of high Vit.E concentration (40%) to P(D,L)LA slowed the coagulation process significantly compared to normal P(D,L)LA at every time point, while a statistically significant difference between absorbance values for P(D,L)LA and 1.0% and 20% Vit.E P(D,L)LA samples was observed only after 50 minutes (p<0.001). However for all Vit.E-enriched P(D,L)LA samples coagulation time was 70-75 minutes (data not shown) indicating an increased thromboresistence compared to the normal P(D,L)LA.

### Statistical analysis

The statistical analysis of data was performed using Graph Pad Prism 2.01 software for Windows and using the Anova test followed by Dunnett's post-hoc test, taking p< 0.05 as the minimum level of significance.

### B) Neointima-like cells adhesion and proliferation

One of the most investigated effects of Vit.E (in particular of the α-tocopherol) is the ability to reduce the rat and human SMC proliferation [29]. As the neointima is a scar tissue derived from hyperproliferation of SMC, the adhesion and proliferation of neointima-like rat cells A10 [30] onto Polylactil-E has been investigated.

Rat clonal cell line A10 (ATCC CRL-1476) was derived from the thoracic aorta of DB1X embryonic rat and possesses many of the properties characteristic of smooth muscle cells.

A10 cells were grown in culture flask (75 cm²) in Dulbecco's modified Eagle's medium (DMEM, Euroclone) supplemented with 10 % heat-inactivated fetal bovine serum (FBS) (Euroclone), penicillin (100 U/ml), streptomycin (100 µg/ml) and L-glutamine (2 mM) (Euroclone) in a humidified atmosphere containing 5% CO₂ at 37°C. Evaluation of A10 cells adhesion and proliferation was performed using a fluorescence microscope Aristoplan Leitz equipped with a digital camera Leica DFC320. A10 cells were seeded onto cell culture grade polystyrene dishes (PS), control (PLA) and Vit.E-enriched P(D,L)LA (PLA10, PLA20 and PLA40) at a concentration of 1.5x10⁴ cells/cm². The number of adherent cells was evaluated after 0.5, 1, 2 and 4 hours incubation while proliferation was estimated counting cells present onto different polymers after 24, 48 and 72 hours.

At the end of incubation time adherent cells were washed three times with cold PBS (pH 7.4) and fixed for 15 minutes at room temperature using a solution of formaldehyde (3.7%) and sucrose (3%) in PBS (pH 7.4). Cells were then stained for 10 minutes at. room temperature in the dark with a 0.2% solution of Acridine Orange (AO) and counted in 10 different fields per sample at 10X magnification. Scoring was performed by three separate observers, blind to the sample treatment using Leika QWin software and express as adherent cells/cm² ± standard deviation (S.D.).

As shown in Fig.7, the number of A10 cells adherent onto PLA, PLA10, PLA20 and PLA40 films increased from 0,5 to 4 hours without statistically significant differences among the different polymers.

In fact, the number of A10 cells ± standard deviation (S.D.) scored onto 1 cm² PLA after 0.5 hours was 275 ± 159 increasing after 2 (1405 ±505) and 3 (855 ± 529) hours. A similar trend was observed for PLA10, 20 and 40 and the number of cells scored onto 1 cm² polymer surface, after 4 hours, was 4,674 ± 1,375 for PLA, 5,040 ± 1, 920 for PLA10, 4, 032 ± 1, 033 for PLA20 and 3,849 ± 916 for PLA40, while cells adhered onto the positive control surface (PS) after 4 hours were 14,204 ± 3,404 (data not shown). A10 cell proliferated onto every polymer but the proliferation kinetics were different (Fig.8). In particular, after 24 hours the cell number scored onto PLA and Vit.E enriched PLA was not different with the exception of PLA20 (p<0.05). After 48 hours all the Vit.E enriched PLA showed a reduction in the proliferation compared to PLA (p<0.05) with the exception of PLA20. The proliferation slowing or arrest was more evident after 72 hours, when cells/cm² ± S.D. scored onto PLA10, 20 and 40 were respectively 110,000 ± 44,400, 136,667 ± 40,000 and 172,222 ± 54,440 (p<0.001 compared to PLA) while a rapid proliferation occurred onto PLA (426,667 ± 31,110 cells/cm²) and, as expected, onto PS surface reaching the number 926,670 ± 60, 000 after 72 hours. No toxic effect on A10 cells (excessive number of floating dead cells) was observed during daily optical inspection performed for all the samples.

Naturally, while the principle of the invention remains the same, the details of construction and the embodiments may widely vary with respect to what has been described and illustrated purely by way of example, without departing from the scope of the present invention as depicted in the appended claims.

### References

1. van der Hoeven BL, Pires NM, Warda HM, Oemrawsingh PV, van Vlijmen BJ, Quax PH, Schalij MJ, van der Wall EE, Jukema JW. Drug-eluting stents: results, promises and problems. Int J Cardiol. 2005;99:9-17.
2. Haery C, Sachar R, Ellis SG. Drug-eluting stents: the beginning of the end of restenosis? Cleve Clin J Med. 2004;71:815-24.
3. van der Giessen WJ, Lincoff AM, Schwartz RS, van Beusekom HM, Serruys PW, Holmes DR Jr, Ellis SG, Topol EJ. Marked inflammatory sequelae to implantation of biodegradable and nonbiodegradable polymers in porcine coronary arteries. Circulation. 1996 ; 94:1690-7.
4. Drachman DE, Edelman ER, Seifert P, Groothuis AR, Bornstein DA, Kamath KR, Palasis M, Yang D, Nott SH, Rogers C. Neointimal thickening after stent delivery of paclitaxel: change in composition and arrest of growth over six months. J Am Coll Cardiol. 2000; 36:2325-32.
5. Suzuki T, Kopia G, Hayashi S, Bailey LR, Llanos G, Wilensky R, Klugherz BD, Papandreou G, Narayan P, Leon MB, Yeung AC, Tio F, Tsao PS, Falotico R, Carter AJ. Stent-based delivery of sirolimus reduces neointimal formation in a porcine coronary model. Circulation. 2001 ;104:1188-93.
6. Lewis AL, Stratford PW. Phosphorylcholine-coated stents. J Long Term Eff Med Implants. 2002;12:231-50.
7. Andreopoulos AG, Hatzi E, Doxastakis M. Synthesis and properties poly(lactic acid). J Mater Sci Mater Med. 1999 Jan;10(1):29-33.
8. Drumright RE, Gruber PR, Henton DE. Polylactic acid technology. Adv Mater 2000, 12 (23):1814-6.
9. Frank RD, Dresbach H, Thelen H, Sieberth HG. Glutardialdehyde induced fluorescence technique (GIFT): a new method for the imaging of platelet adhesion on biomaterials. J Biomed Mater Res. 2000; 52:374-81.
10. Peltroche-Llacsahuanga H, Schmidt S, Schnitzler N, Lutticken R, Haase G. Simultaneous measurement of biopolymer-mediated Mac-1 up-regulation and adherence of neutrophils: a novel flow cytometric approach for predicting initial inflammatory interaction with foreign materials. J Immunol Methods. 2001; 258:13-25.
11. Vogt F, Stein A, Rettemeier G, Krott N, Hoffmann R, vom Dahl J, Bosserhoff AK, Michaeli W, Hanrath P, Weber C, Blindt R. Long-term assessment of a novel biodegradable paclitaxel-eluting coronary polylactide stent. Eur Heart J. 200-4; 25:1330-40.
12. Al Suwaidi J, Berger PB, Holmes DR Jr. Coronary artery stents. JAMA 2000; 284:1828-1836.
13. Mintz GS, Popma JJ, Pichard AD, Kent KM, Satler LF, Wrong C, Hong MK, Kovach JA, Leon MB. Arterial remodeling after coronary angioplasty: a serial intravascular ultrasound study. Circulation 1996; 94:35-43.
14. Hoffmann R, Mintz GS, Dussaillant GR, Popma JJ, Pichard AD, Satler LF, Kent KM, Griffin J, Leon MB. Patterns and mechanisms of in-stent restenosis. A serial intravascular ultrasound study. Circulation 1996; 94:1247-1254.
15. Lowe HC, Oesterle SN, Khachigian LM. Coronary in-stent restenosis: current status and future strategies. J Am Coll Cardiol 2002; 39:183-193.
16. Vogt F, Stein A, Rettemeier G, Krott N, Hoffmann R, vom Dahl J, Bosserhoff AK, Michaeli W, Hanrath P, Weber C, Blindt R. Long-term assessment of a novel biodegradable paclitaxel-eluting coronary polylactide stent. Eur Heart J 2004; 25:1330-1340.
17. Drachman DE, Edelman ER, Seifert P, Groothuis AR, Bornstein DA, Kamath KR, Palasis M, Yang D, Nott SH, Rogers C. Neointimal thickening after stent delivery of paclitaxel: change in composition and arrest of growth over six months. J Am Coll Cardiol 2000; 36:2325-2332.
18. Moses JW, Leon MB, Popma JJ, Fitzgerald PJ, Holmes DR, O'Shaughnessy C, Caputo RP, Kereiakes DJ, Williams DO, Teirstein PS, Jaeger JL, Kuntz RE; SIRIUS Investigators. Sirolimus-eluting stents versus standard stents in patients with stenosis in a native coronary artery. N Engl J Med 2003; 349:1315-1323.
19. Brigelius-Flohe R, Kelly FJ, Salonen JT, Neuzil J, Zingg JM, Azzi A. The European perspective on vitamin E: current knowledge and future research. Am J Clin Nutr. 2002; 76:703-16.
20. Zingg JM, Azzi A. Non-antioxidant activities of vitamin E. Curr Med Chem 2004;11:1113-33.
21. Devaraj S, Li D, Jialal I. The effects of alpha tocopherol supplementation on monocyte function. Decreased lipid oxidation, interleukin 1 beta secretion, and monocyte adhesion to endothelium. J Clin Invest. 1996; 98:756-63.
22. Cachia O, Benna JE, Pedruzzi E, Descomps B, Gougerot-Pocidalo MA, Leger CL. Alpha-tocopherol inhibits the respiratory burst in human monocytes. Attenuation of p47(phox) membrane translocation and phosphorylation. J Biol Chem. 1998; 273:32801-5.
23. Mendez JA, Aguilar MR, Abraham GA, Vazquez B, Dalby M, Di Silvio L, San Roman J. New acrylic bone cements conjugated to vitamin E: curing parameters, properties, and biocompatibility. J Biomed Mater Res. 2002; 62:299-307.
24. Schubert MA, Wiggins MJ,· DeFife KM, Hiltner A, Anderson JM. Vitamin E as an antioxidant for poly(etherurethane urea): in vivo studies. J Biomed Mater Res. 1996; 32:493-504.
25. Sasaki M, Hosoya N, Saruhashi M. Vitamin E modified cellulose membrane. Artif Organs. 2000; 24:779-89.
26. Tsuruoka S, Kawaguchi A, Nishiki K, Hayasaka T, Fukushima C, Sugimoto K, Saito T, Fujimura A. Vitamin E-bonded hemodialyzer improves neutrophil function and oxidative stress in patients with end-stage renal failure. Am J Kidney Dis. 2002; 39:127-33.
27. Boyum A. Isolation of mononuclear cells and granulocytes from human blood. Scand J Clin Lab Invest 1968; 97: 77-89.
28. Imai Y, Nose Y. A new method for evaluation of antithrombogenicity of materials. J Biomed Mater Res 1972; 6:165-72.
29. Azzi A, Boscoboinik D, Clement S, Marilley D, Ozer NK, Ricciarelli R, Tasinato A. Alpha-tocopherol as a modulator of smooth muscle cell proliferation. Prostaglandins Leukot Essent Fatty Acids 1997; 57:507-514.
30. Rao RS, Miano JM, Olson EN, Seidel CL. The A10 cell line: a model for neonatal, neointimal, or differentiated vascular smooth muscle cells? Cardiovasc Res. 1997; 36:118-126.

## Claims

1. Use of a biodegradable polylactic acid polymer for coating implantable prosthesis, **characterized in that** the polymer is constituted by poly(D,L)lactic acid and α-tocopherol, and **in that** at least part of the poly(D,L)lactic acid molecules are bound to at least part of the α-tocopherol molecules.

2. Use according to claim 1, wherein α-tocopherol is present in an amount comprised between 10 and 40% by weight with respect to the total weight of the polymer, preferably between 10 and 20% by weight with respect to the total weight of the polymer.

3. Use according to claim 1, wherein α-tocopherol is present in an amount comprised between 30 and 40% by weight with respect to the total weight of the polymer.

4. Use according to any one of claims 1 to 3, wherein the polymer has a water contact angle comprised between 45° and 70°, preferably between 49° and 60°, when distilled water dropped on the polymer surface.

5. Use according to any one of claims 1 to 4, wherein the polymer has a protein adsorption higher than 150 µg/cm², preferably higher than 200 µg/cm², more preferably higher than 300 µg/cm², when human plasma is incubated on the polymer surface at 37°C.

6. Use according to any one of claims 1 to 5, wherein the polymer has a first glass transition temperature higher than 25°C, preferably higher than 30°C.

7. Use according to any one of claims 1 to 6, wherein the polymer has a second glass transition temperature comprised between -40° and -50°C, preferably about -46°C.

8. Use according to any one of claims 1 to 7, wherein platelet adhesion on the polymer surface measured as the percentage of area covered by adherent platelet on the polymer surface is lower than 36%, preferably about 4%, when platelets are seeded and incubated on the polymer surface at 37°C.

9. Use according to any one of claims 1 to 8, wherein granulocyte adhesion on the polymer surface measured as granulocyte cell/cm² on the polymer surface is lower than 400,000 cell/cm², preferably lower than 20,000 cell/cm², when granulocytes are seeded and incubated on the polymer surface at 37°C.

10. Use according to any one of claims 1 to 9, wherein the polymer is able to induce blood clotting after a period of contact between blood and the polymer surface of 75 minutes.

11. Use according to any one of claims 1 to 10, wherein the polymer is applied on the implantable prosthesis by spraying with or dipping in a polymer solution the prosthesis.

12. Use according to claim 11, wherein the polymer solution is obtained by i) dissolving poly(D,L)lactic acid in a first solvent obtaining a first solution, ii) dissolving α-tocopherol in a second solvent obtaining a second solution, iii) mixing the first and the second solution, thus obtaining the polymer solution.

13. Use according to claim 12, wherein the first solvent is selected from chloroform, and the second solvent is selected from ethanol.

14. Use according to any one of claims 11 to 13, wherein the phase of spraying with or dipping in the polymer solution the prosthesis is carried out at least once, preferably more than twice.

15. Use according to any one of claims 11 to 14, wherein after the phase of spraying with or dipping in the polymer solution the prosthesis, the solvents of the polymer solution are allowed to evaporate, thus forming a polymer film on the prosthesis surface.

16. Use according to any one of claims 1 to 15, wherein the polymer acts as a drug delivery system.

17. Use according to any one of claims 1 to 16, wherein the implantable prosthesis is selected from, a stent, a stent graft, a synthetic vascular graft, a heart valve, a catheter, a vascular prosthetic filter, a pacemaker, a pacemaker lead, a defibrilator, a septal closure device, a vascular clip, a vascular aneurysm occluder, a hemodialysis graft, a hemodialysis catheter, an atrioventricular shunt, an aortic aneurysm graft device, a venous valve, a suture, a vascular anastomosis clip, an indwelling venous catheter, an indwelling arterial catheter, a vascular sheath and a drug delivery port.

## Patentansprüche

1. Verwendung eines biologisch abbaubaren Polymilchsäurepolymers zum Beschichten einer implantierbaren Prothese, **dadurch gekennzeichnet, dass** das Polymer durch Poly(D,L)-Milchsäure und α-Tocopherol gebildet ist, und dass zumindest ein Teil der Poly(D,L)-Milchsäuremoleküle an zumindest einen Teil der α-Tocopherolmoleküle gebunden ist.

2. Verwendung nach Anspruch 1, wobei α-Tocopherol in einer Menge vorliegt, die zwischen 10 und 40 Gew.-% in Bezug auf das Gesamtgewicht des Polymers, vorzugsweise zwischen 10 und 20 Gew.-% in Bezug auf das Gesamtgewicht des Polymers liegt.

3. Verwendung nach Anspruch 1, wobei α-Tocopherol in einer Menge vorliegt, die zwischen 30 und 40 Gew.-% in Bezug auf das Gesamtgewicht des Polymers liegt.

4. Verwendung nach einem der Ansprüche 1 bis 3, wobei das Polymer einen Wasserkontaktwinkel aufweist, der zwischen 45° und 70°, vorzugsweise zwischen 49° und 60° liegt, wenn destilliertes Wasser auf die Polymeroberfläche tropft.

5. Verwendung nach einem der Ansprüche 1 bis 4, wobei das Polymer eine Proteinadsorption aufweist, die höher als 150 µg/cm², vorzugsweise höher als 200 µg/cm², stärker bevorzugt höher als 300 µg/cm² ist, wenn menschliches Plasma auf der Polymeroberfläche bei 37°C inkubiert wird.

6. Verwendung nach einem der Ansprüche 1 bis 5, wobei das Polymer eine erste Glasübergangstemperatur aufweist, die höher als 25°C, vorzugsweise höher als 30°C ist.

7. Verwendung nach einem der Ansprüche 1 bis 6, wobei das Polymer eine zweite Glasübergangstemperatur aufweist, die zwischen -40°C und -50°C, vorzugsweise bei etwa -46°C liegt.

8. Verwendung nach einem der Ansprüche 1 bis 7, wobei das Plättchenhaftvermögen auf der Polymeroberfläche, gemessen als Prozentanteil der durch auf der Polymeroberfläche anhaftende Plättchen bedeckten Fläche, geringer ist als 36%, vorzugsweise etwa 4% ist, wenn Plättchen auf der Polymeroberfläche bei 47°C gekeimt und inkubiert werden.

9. Verwendung nach einem der Ansprüche 1 bis 8, wobei das Granulozytenhaftvermögen auf der Polymeroberfläche, gemessen als Granolytenzellen/cm² auf der Polymeroberfläche, geringer ist als 400.000 Zellen/cm², vorzugsweise geringer als 20.000 Zellen/cm² ist, wenn Granulozyten auf der Polymeroberfläche bei 37°C gekeimt und inkubiert werden.

10. Verwendung nach einem der Ansprüche 1 bis 9, wobei das Polymer in der Lage ist, nach einer 75-minütigen Kontaktzeit zwischen Blut und der Polymeroberfläche eine Blutgerinnung herbeizuführen.

11. Verwendung nach einem der Ansprüche 1 bis 10, wobei das Polymer auf die implantierbare Prothese aufgebracht wird, indem die Prothese mit einer Polymerlösung besprüht oder in diese eingetaucht wird.

12. Verwendung nach Anspruch 11, wobei die Polymerlösung erhalten wird, indem i) Poly(D,L)-Milchsäure unter Erhalt einer ersten Lösung in einem ersten Lösungsmittel gelöst wird, ii) α-Tocopherol unter Erhalt einer zweiten Lösung in einem zweiten Lösungsmittel gelöst wird, iii) die erste und die zweite Lösung gemischt werden, wodurch die Polymerlösung erhalten wird.

13. Verwendung nach Anspruch 12, wobei das erste Lösungsmittel ausgewählt ist aus Chloroform und das zweite Lösungsmittel ausgewählt ist aus Ethanol.

14. Verwendung nach einem der Ansprüche 11 bis 13, wobei die Phase des Besprühens der Prothese mit der oder des Eintauchens selbiger in die Polymerlösung mindestens einmal, vorzugsweise mehr als zweimal durchgefiihrt wird.

15. Verwendung nach einem der Ansprüche 11 bis 14, wobei man die Lösungsmittel der Polymerlösung nach der Phase des Besprühens der Prothese mit der oder des Eintauchens selbiger in die Polymerlösung verdampfen lässt, wodurch ein Polymerfilm auf der Prothesenoberfläche gebildet wird.

16. Verwendung nach einem der Ansprüche 1 bis 15, wobei das Polymer als Arzneistoffabgabesystem fungiert.

17. Verwendung nach einem der Ansprüche 1 bis 16, wobei die implantierbare Prothese ausgewählt ist aus einem Stent, einem Stent-Implantat, einem synthetischen Gefäßersatz, einer Herzklappe, einem Katheter, einem Gefäßprothesefilter, einem Schrittmacher, einer Schrittmacherelektrode, einem Defibrillator, einer Septumverschlussvorrichtung, einer Gefäßklammer, einem Gefäßaneurysma-Occluder, einem Hämodialyse-Implantat, einem Hämodialysekatheter, einem Atrioventrikular-Shunt, einer Aortaaneurysmatransplantatvorrichtung, einer Venenklappe, einer Wundnaht, einer Gefäßanastomoseklemme, einem Dauervenenkatheter, einem Dauerarterienkatheter, einer Gefäßscheide und einem Arzneistoffabgabe-Port.

## Revendications

1. Utilisation d'un polymère d'acide polylactique biodégradable pour revêtir une prothèse implantable, **caractérisée en ce que** le polymère est constitué d'acide poly(D,L)lactique et d'α-tocophérol, et **en ce qu'**au moins une partie des molécules d'acide poly(D,L)lactique sont liées à au moins une partie des molécules d' α-tocophérol.

2. Utilisation selon la revendication 1, dans laquelle l' α-tocophérol est présent en une quantité comprise entre 10 et 40 % en poids par rapport au poids total du polymère, de préférence entre 10 et 20 % en poids par rapport au poids total du polymère.

3. Utilisation selon la revendication 1, dans laquelle l' α-tocophérol est présent en une quantité comprise entre 30 et 40 % en poids par rapport au poids total du polymère.

4. Utilisation selon l'une des revendications 1 à 3, dans laquelle le polymère présente un angle de contact avec l'eau compris entre 45° et 70°, de préférence entre 49° et 60°, lorsqu'une goutte d'eau distillée est déposée sur la surface du polymère.

5. Utilisation selon l'une des revendications 1 à 4, dans laquelle le polymère présente une adsorption des protéines supérieure à 150 µg/cm², de préférence supérieure à 200 µg/cm², très préférentiellement supérieure à 300 µg/cm², lorsque du plasma humain est incubé sur la surface du polymère à 37°C.

6. Utilisation selon l'une des revendications 1 à 5, dans laquelle le polymère présente une température de première transition vitreuse supérieure à 25°C, de préférence supérieure à 30°C.

7. Utilisation selon l'une des revendications 1 à 6, dans laquelle le polymère présente une température de seconde transition vitreuse comprise entre -40 et -50°C, de préférence environ -46°C.

8. Utilisation selon l'une des revendications 1 à 7, dans laquelle l'adhésion des plaquettes sur la surface du polymère mesurée par le pourcentage de l'aire recouverte par les plaquettes adhérentes sur la surface du polymère est inférieure à 36 %, de préférence environ 4 %, lorsque des plaquettes sont semées et incubées sur la surface du polymère à 37°C.

9. Utilisation selon l'une des revendications 1 à 8, dans laquelle l'adhésion des granulocytes sur la surface du polymère mesurée en cellules de granulocytes/cm² sur la surface du polymère est inférieure à 400 000 cellules/cm², de préférence inférieure à 20 000 cellules/cm², lorsque des granulocytes sont semés et incubés sur la surface du polymère à 37°C.

10. Utilisation selon l'une des revendications 1 à 9, dans laquelle le polymère est capable d'induire la coagulation du sang après une période de contact de 75 minutes entre le sang et la surface du polymère.

11. Utilisation selon l'une des revendications 1 à 10, dans laquelle le polymère est appliqué sur la prothèse implantable par pulvérisation d'une solution de polymère sur la prothèse, ou trempage dans une solution de polymère.

12. Utilisation selon la revendication 11, dans laquelle la solution de polymère est obtenue par i) dissolution de l'acide poly(D,L)lactique dans un premier solvant pour obtenir une première solution, ii) dissolution de l'α-tocophérol dans un second solvant pour obtenir une seconde solution, iii) mélange de la première et de la seconde solution, pour obtenir ainsi la solution de polymère.

13. Utilisation selon la revendication 12, dans laquelle le premier solvant est choisi parmi le chloroforme, et le second solvant est choisi parmi l'éthanol.

14. Utilisation selon l'une des revendications 11 à 13, dans laquelle la phase de pulvérisation de la solution de polymère sur la prothèse, ou de trempage dans la solution de polymère, est exécutée au moins une fois, de préférence plus de deux fois.

15. Utilisation selon l'une des revendications 11 à 14, dans laquelle après la phase de pulvérisation de la solution de polymère sur la prothèse, ou de trempage dans la solution de polymère, on laisse s'évaporer les solvants de la solution de polymère, de manière à former ainsi un film de polymère sur la surface de la prothèse.

16. Utilisation selon l'une des revendications 1 à 15, dans laquelle le polymère joue un rôle de système de délivrance de médicament.

17. Utilisation selon l'une des revendications 1 à 16, dans laquelle la prothèse implantable est choisie parmi : un stent, un stent-greffe, une greffe vasculaire synthétique, une valvule cardiaque, un cathéter, un filtre prothétique vasculaire, un stimulateur cardiaque, une sonde de stimulateur cardiaque, un défibrillateur, un dispositif de fermeture septale, un clip vasculaire, un dispositif d'occlusion d'anévrysme vasculaire, une greffe d'hémodialyse, un cathéter d'hémodialyse, un pontage atrioventriculaire, un dispositif de greffe d'anévrysme aortique, une valvule veineuse, une suture, un clip d'anastomose vasculaire, un cathéter veineux posé à demeure, un cathéter artériel posé à demeure, une gaine vasculaire et un orifice de délivrance de médicament.
